# EUROPEAN PATENT APPLICATION

(11) **EP 1 848 073 A1**
(43) Date of publication of application: **24.10.2007**
(21) Application number: 06112776.7
(22) Date of filing: 19.04.2006
(51) Int. Cl.: H01S 3/23, H01S 3/102, H01S 3/0941

(54) **Switchable laser device and method for operating said device**

(71) Applicant: Multitel ASBL, 7000 Mons (BE)
(72) Inventor: Hernandez, Yves, 59570, TAISNIERES SUR HON (FR); Vercambre, Clément, 59750, FEIGNIES (FR)
(74) Representative: pronovem

(57) **Abstract**

The present invention relates to a laser device (1) comprising an active element (3) for emitting light and a tuneable pumping source (5) arranged for providing energy to said active element (3). The laser device is arranged for emitting light with a first set of characteristics derivable from the active element. The laser device is further arranged for emitting light with a second set of characteristics derivable from the tuneable pumping source (5).

## Description

### Field of the invention

The present invention relates to the field of laser devices comprising an active light emitting element and a tuneable pumping source for providing energy to the active element.

### State of the art

First lasers appeared in the 1970s. Since this technological breakthrough a lot of new applications and possibilities have appeared beginning by improvements in the field of medicine. Due to cost issues a lot of technological work has been performed to optimise the laser structures and to render the devices as polyvalent as possible. In the objective of reducing cost and increasing the efficiency of such devices, the fibre laser has appeared in the 1990s. This emerging technology permits to realise more reliable, compact and cost effective laser equipment. Furthermore a lot of work has been done to extend the laser performance, such that a wider range of applications can be addressed. Optical parametric oscillators, for instance, allow switching the various wavelength spectra wherein a laser device emits, thus making a single device usable for various applications, thereby reducing the investment cost for the final end-user.

Certain applications such as industrial marking and cutting, medical treatment and cutting or chemical detection require different types of lasers. Detection applications often require sources that are wavelength tuneable over at least a few nm range (see patent document WO 95/34007-A1). Marking applications require the possibility to adapt the laser spot to the dimensions of the patterns to be written. For medical applications, such as dermatology, the manufacturers propose devices emitting at different wavelength regions in order to treat different diseases with a single device.

Document WO 95/34007-A1 discloses a laser device tuneable over a few nanometres into a given wavelength emission window. In that case, tuneability is obtained by moving a grating behind optical fibres. However, such a limited tuneable range obviously does not substantially change the device characteristics and does not yield an extended field of application.

Patent application WO 02/22033-A1 relates to a laser device able to switch between two wavelengths in a mechanical way in order to provide the user with an equipment having extended capabilities. However, the proposed solution is complex and expensive. No other features than wavelength switching are added to the laser device.

WO 02/075976-A1 teaches a thermally tuneable way to optimise the optical gain in a fibre amplifier. The invention uses this kind of thermal regulation of the pump diode. The thermal switching of the pump is used for the stabilisation and the optimisation of the laser.

### Aims of the invention

The present invention aims to provide a laser device capable of emitting light having characteristics that can be switched between at least two sets of characteristics. In another aspect the invention also relates to a method for operating such a laser device.

### Summary of the invention

The present invention relates to a laser device comprising an active element for emitting light and a tuneable pumping source arranged for providing energy to said active element, whereby the laser device is arranged for emitting light with a first set of characteristics derivable from the active element. The laser device is further arranged for emitting light with a second set of characteristics derivable from the tuneable pumping source.

In a preferred embodiment the laser device is further arranged for emitting light having characteristics that are a combination of said first and second set of characteristics.

Advantageously the active element is a doped fibre. Alternatively the active element is a solid-state element, a gas element or a dye element.

In another embodiment the tuneable pumping source is a laser diode.

Preferably the first and second set of characteristics comprise at least one parameter from the group of parameters {wavelength, light beam quality, pulse duration}.

In a second aspect the invention relates to a method for switching between operable states of a laser device comprising the steps of :
- providing a laser device comprising an active element and a tuneable pumping source,
- emitting with the laser device light with a first set of characteristics, said characteristics derived from the active element or the tuneable pumping source,
- changing the emitted light characteristics from the first set into a second set of characteristics derived from the active element or the tuneable pumping source,
- emitting with the laser device light with the second set of characteristics.

In a preferred embodiment either the first or the second set of characteristics is derived from a combination of characteristics derived from the active element or the tuneable pumping source.

In a specific embodiment the step of changing is performed by tuning the temperature of the tuneable pumping source.

### Short description of the drawings

Fig. 1 represents a general scheme of a laser device with an active element.

Fig. 2 represents a general scheme of a fibre laser device comprising an active element and a pumping laser as tuneable pumping source.

Fig. 3 represents the switching of the output light between two sets of characteristics by adjusting the temperature of the pumping source.

Fig. 4 represents three examples. In Fig.4A wavelength tuning of a fibre laser is illustrated. Fig. 4B shows laser beam quality tuning of a double clad doped fibre. Fig.4C shows the pulse tuning of an amplifier system.

Fig. 5 represents a design with an improved extinction ratio.

Fig. 6 represents a way to work on the active element to limit the absorption coefficient out of the absorption peak.

### Detailed description of the invention

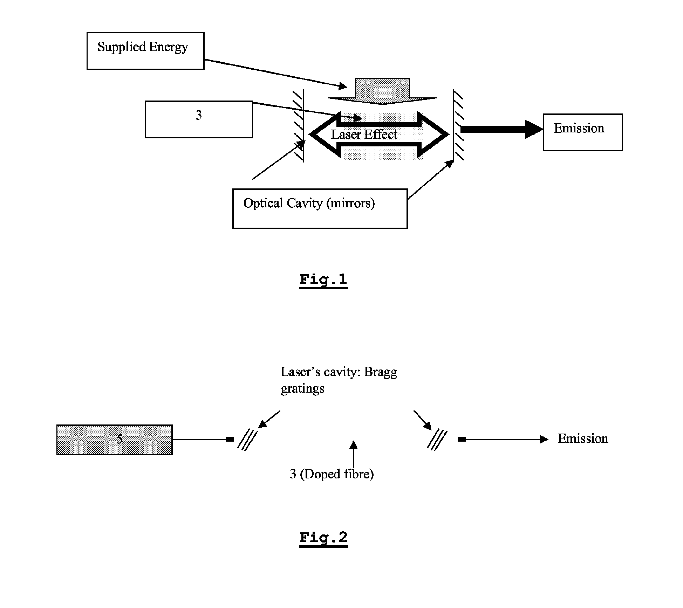

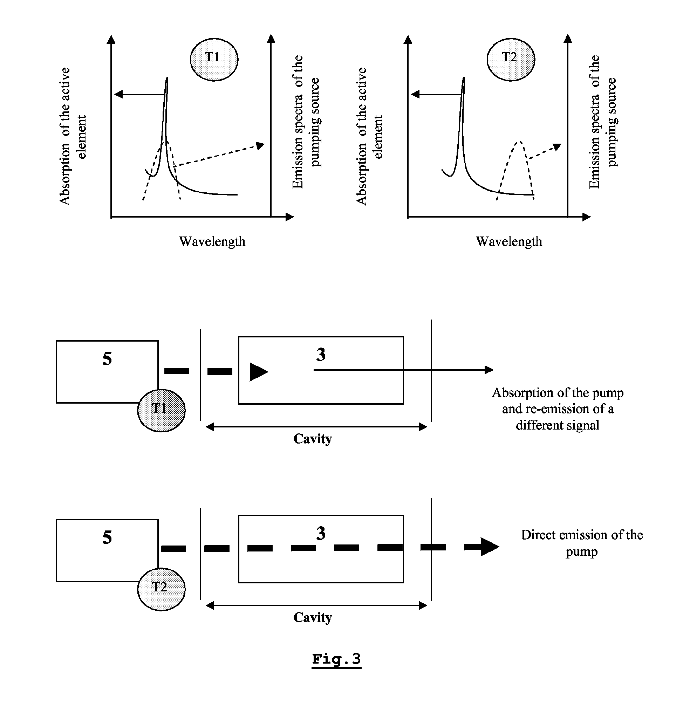

For the active element (3) of a laser device (1) to be operable, it must be supplied with energy. This energy is then converted and emitted by the active element of the device and the laser radiation appears (Fig.1). In certain cases (e.g. for fibre lasers), the supplied energy can come from a tuneable pumping source (5), which often is a laser diode pump. Then the active element (3) of the laser device (1) absorbs the incident light and re-emits a laser radiation. The wavelength of this radiation is determined by the emission spectrum of the active element (Fig.2). Then the active element (3) of the laser device absorbs the incident light and re-emits a laser radiation. In many prior art publications measures are taken in order to keep the light of the pumping source at a wavelength corresponding to an absorption peak of the active element.

In order to obtain a highly versatile laser device, the present invention proposes a laser device and method for operating such device allowing switching at least between the emitted radiation of the active element (3) and the pumping radiation of the tuneable pumping source (5). The characteristics set by the active element (3) on the one hand and the tuneable laser source (5) on the other hand define two extremes. It is clear that also intermediate sets of characteristics can be obtained, as the transition between the two extremes occurs in a continuous fashion. For example, when tuning the light output by the laser device can have a set of characteristics that are partially derived from the active element and partially from the tuneable laser source. The invention also allows having cost effective laser devices with more options in terms of output light properties.

The invention is further explained for the case of fibre lasers. However, this is no limitation to the scope of the invention, as the concept is equally compatible with other types of lasers (solid-state, gas, dye...) .

In a fibre laser or amplifier, the pumping laser diode wavelength is generally selected to match an absorption peak of the doped fibre spectra. Then the pumping signal is absorbed by the fibre and re-emitted at a different wavelength depending on the nature of the doping elements into the fibre. By tuning the pumping diode wavelength, it is then possible to move the pumping signal out of the absorption spectra of the doped fibre. The pumping light is in that case no longer absorbed and is mainly emitted at the laser device output, while the original signal generated by the doped fibre is considerably decreased. Then the output signal of the fibre laser or amplifier can be switched between the pumping signal and the light emission of the doped fibre. It is then possible to switch between two wavelength emission regions for instance.

In Fig.3 T1 denotes the pumping source temperature at which the pumping source emission matches the absorption peak of the active element of the laser device. T2 represents a temperature where the emission peak of the pumping source is shifted to.

An example is given for a double clad doped fibre laser, wherein the doped fibre is the active element of the laser and a laser diode provides the pumping light.

In the case of an amplifier, it is possible to switch in a similar way between the pumping light and the amplified signal. Consider the case of a pumping diode that can operate continuously or at a low repetition rate and a seed signal laser (i.e. input laser signal) providing a high speed pulsed emission. It is again possible to switch between these two modes of operation by tuning the pumping light. So it is possible to develop a single device that will operate over different pulse mode regimes.

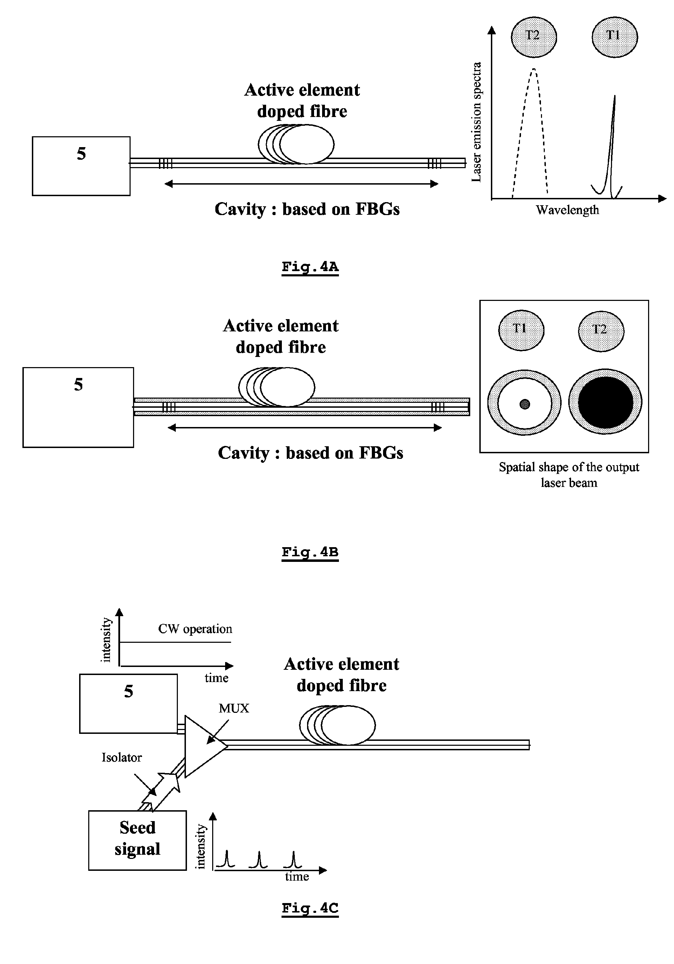

Fig.4 shows three examples. Example 1 relates to wavelength tuning of a fibre laser (e.g. based on an Erbium doped fibre or a co-doped Erbium-Ytterbium fibre, but also other doping elements are usable). The pumping diode has an emission wavelength of 976nm, corresponding to a maximum peak absorption of Erbium. By tuning for instance the diode temperature from T1 to T2, it is possible to shift its wavelength away from the absorption maximum of the fibre. Then the pump signal propagates through the whole fibre laser structure without being absorbed or only minimally absorbed and is emitted instead of the 1,55µm original signal. In this way a double wavelength emitting laser device can be obtained. This laser is operable in two very different wavelength regions: around 1µm and around 1.55µm. It can be applied in very different applications as surgery and dermatology in the case of medical applications. Indeed the 1.55µm wavelength matches an absorption peak of water, whereas the 1µm region is well absorbed by melanin, these two elements both being present in the human skin.

In Example 2 beam quality tuning is illustrated for the case of a double clad doped fibre. A double clad Ytterbium doped fibre is considered, again pumped by a 976nm laser diode. When no tuning is applied to the pumping diode, the radiation is absorbed by the Ytterbium doping element in the fibre. Next, the radiation is converted into a 1µm single-mode signal, as the Ytterbium is mainly present in the single-mode core of the double clad fibre. However, when the pumping radiation wavelength is tuned, it is no longer absorbed by the doped core of the fibre. It can then propagate through the multi-mode core of the double clad fibre. Consequently, the laser device output is tuned from a single-mode operation to a multi-mode operation. In this example again a spectral shift is obtained, but, more importantly, the device is given a new feature in that it is capable of switching between two spatial beam qualities. By applying a continuous tuning method, the tuning can be made progressive. The operator then has the option to select the laser beam quality according to the patterns he wants to realise. This is of particular interest in the case of material processing as for instance in laser marking.

Example 3 illustrates the pulse tuning of an amplifier system. A double clad or single-mode amplifier is considered. Again, by tuning the wavelength emission of the light from the pumping source it is possible to shift it away from the absorption spectra of the active element. This is always the basic principle underlying the invention. As a consequence, if the seed signal of the amplifier is operating in a high speed pulse mode, the pumping signal is matched with the absorption spectra of the amplifying media, then the seed high speed light will be amplified through the system and emitted by the device. Now the seed source is turned off and the pumping light is shifted away from the absorption spectra. Then the device will emit the continuous wave light generated by the pump. And if the pump is driven by a sweep generator this signal can be also pulsed at lower rate than the high speed source. Then the pulse repetition rate and duration will possibly be tuned over a larger range with this method.

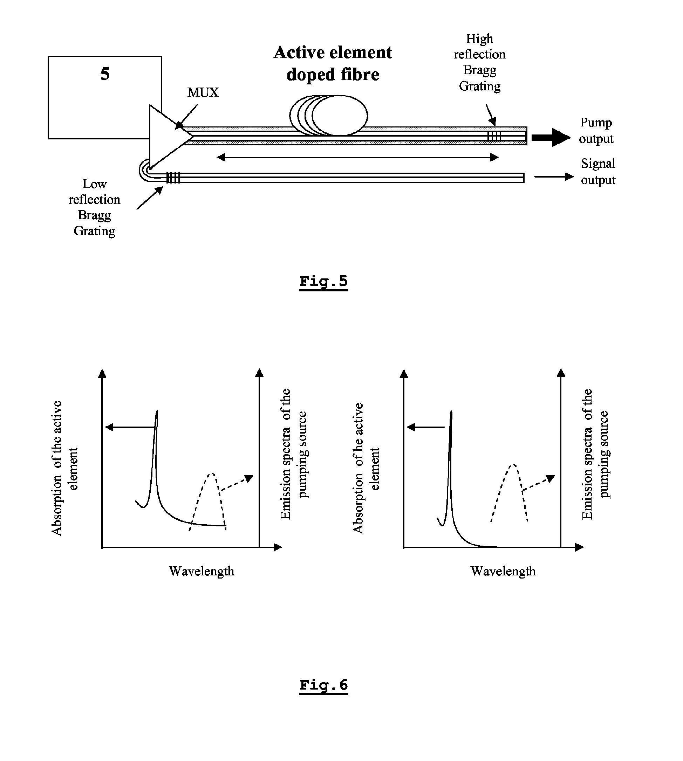

Fig.5 shows an improvement of the design in order to have a better extinction ratio (the ratio of emitted light from the second characteristic to emitted light from the first one) between the two wavelengths.

Fig.6 shows a further embodiment of the invention by working on the active element in order to limit the absorption coefficient out of the absorption peak. The invention can be improved by optimising the absorption spectra of the gain medium, for instance a sharper absorption peak of the doped fibre can help to have a switch with a lower required tuning range for the pump.

Other possible improvements of the invention can be envisaged. Again, as already explained, the principle of invention is to switch laser or amplifier properties by tuning its pumping wavelength out of the absorption peak of the active element absorption band. This is applicable to fibre based lasers and amplifier but also to solid-state, dye or gas... pumped by another optical source. As well an optimised pump system, with high wavelength dependency with temperature or combining Bragg grating and thermal effect could also be an improvement of this invention.

## Claims

1. Laser device (1) comprising an active element (3) for emitting light and a tuneable pumping source (5) arranged for providing energy to said active element (3), said laser device being arranged for emitting light with a first set of characteristics derivable from said active element, **characterised in that** the laser device is further arranged for emitting light with a second set of characteristics derivable from said tuneable pumping source (5).

2. Laser device as in claim 1, further arranged for emitting light having characteristics that are a combination of said first and said second set of characteristics.

3. Laser device as in any of claims 1 to 5, wherein said first and second set of characteristics comprise at least one parameter from the group of parameters {wavelength, light beam quality, pulse duration}.

4. Laser device as in claim 1, 2 or 3, wherein said active element (3) is a doped fibre.

5. Laser device as in claim 1, 2 or 3, wherein said active element (3) is a solid-state element, a gas element or a dye element.

6. Laser device as in any of claims 1 to 5, wherein said tuneable pumping source (5) is a laser diode.

7. Amplifier comprising a laser device as in any of claims 1 to 6.

8. Method for switching between operable states of a laser device comprising the steps of :
- providing a laser device (1) comprising an active element (3) and a tuneable pumping source (5),
- emitting with said laser device (1) light with a first set of characteristics, said characteristics derived from said active element (3) or said tuneable pumping source (5),
- changing the emitted light characteristics from said first set into a second set of characteristics derived from said active element (3) or said tuneable pumping source (5),
- emitting with said laser device (1) light with said second set of characteristics.

9. Method as in claim 8, wherein either said first or said second set of characteristics is derived from a combination of characteristics derived from said active element (3) or said tuneable pumping source (5).

10. Method as in claim 8 or 9, wherein said step of changing is performed by tuning the temperature of said tuneable pumping source (5).
